(19) Europäisches Patentamt

European Patent Office

Office européen des brevets

(11) Veröffentlichungsnummer: **0 538 739 B1**

(12) **EUROPÄISCHE PATENTSCHRIFT**

(45) Veröffentlichungstag der Patentschrift: **21.12.94**

(21) Anmeldenummer: **92117635.0**

(22) Anmeldetag: **15.10.92**

�milnt. Cl.⁵: **A61B 10/00**, A61B 5/05, A61B 5/103, A61H 39/00

(54) **Vorrichtung zur Bestimmung des Gesundheitszustandes eines Lebewesens.**

(30) Priorität: **23.10.91 DE 4134960**

(43) Veröffentlichungstag der Anmeldung:
**28.04.93 Patentblatt 93/17**

(45) Bekanntmachung des Hinweises auf die
Patenterteilung:
**21.12.94 Patentblatt 94/51**

(84) Benannte Vertragsstaaten:
**AT BE CH DE DK ES FR GB GR IE IT LI LU MC NL PT SE**

(56) Entgegenhaltungen:
**WO-A-88/05283        WO-A-88/08273
CH-A- 448 372          DE-A- 2 719 341
FR-A- 2 236 514        FR-A- 2 418 646
US-A- 3 939 841        US-A- 4 310 003**

(73) Patentinhaber: **Reinhard, Max
Kirschblütenweg 7
D-61348 Bad Homburg (DE)**

(72) Erfinder: **Popp, Fritz-Albert
Opelstrasse 10
W-6750 Kaiserslautern (DE)**

(74) Vertreter: **Schmidt, Horst, Dr.
H. Schmidt & B. Müller,
Postfach 44 01 20
D-80750 München (DE)**

## Beschreibung

Die Erfindung betrifft ein Verfahren und eine Vorrichtung zur Ermöglichung einer Aussage über den Gesundheitszustand eines Lebewesens anhand eines Vergleiches einer ausgewählten physiologischen Kenngrösse des Lebewesens mit einer entsprechenden für den gesunden Zustand kennzeichnenden Referenzkenngrösse.

Die Erfindung betrifft insbesondere ein Verfahren und eine Vorrichtung zur Ermöglichung einer Aussage über den ganzheitlichen Gesundheitszustand von Mensch oder Tier.

Alle Geräte, die in der medizinischen Diagnostik eingesetzt werden, erfassen eine bestimmte Kenngrösse oder einen bestimmten Parameter eines Patienten, z.B. Pulsfrequenz, Blutdruck, chemische Zusammensetzung des Blutes etc. Da die Normbereiche aus entsprechenden Messwerten einer gesunden Population bekannt sind, kann aus der Abweichung der Istwerte von der Norm ein Kriterium für die Art und Schwere einer Erkrankung ermittelt werden. Der Befund wird aus einer Vielzahl verschiedener Kenngrössen erhoben, wobei von Fall zu Fall die medizinische Erfahrung für die Auswahl der jeweiligen Kenngrössen ausschlaggebend ist. Es ist jedoch bislang nicht gelungen, auch nicht mit "alternativen" Methoden der sog. "Ganzheitsmedizin", eindeutige und objektive Kriterien für den "ganzheitlichen" Zustand eines Patienten zu ermitteln.

Der Erfindung liegt demzufolge die Aufgabe zugrunde, ein Verfahren und eine Vorrichtung der eingangs erwähnten Gattung zu schaffen, die eine zuverlässige Aussage über den ganzheitlichen Gesundheitszustand eines Probanden ermöglichen. Ausserdem soll es die Erfindung ermöglichen festzustellen, in welchem Masse das Befinden des Probanden insgesamt von einem Idealzustand abweicht. Die Vorrichtung soll ferner die wirtschaftliche Voraussetzung für die Prüfung einer grossen Anzahl von Probanden erlauben, indem sie schnell und preisgünstig vorgenommen werden kann.

Das erfindungsgemässe Verfahren zeichnet sich durch die Merkmale im kennzeichnenden Teil des Patentanspruches 1 aus. Die ausgewählte physiologische Kenngrösse wird somit an einer statistisch signifikanten Vielzahl von über einen bestimmten Flächenbereich des Lebewesens verteilten Messstellen erfasst und die Auswertung mittels Methoden der Statistik anhand der Häufigkeitsverteilung der erfassten Messwerte mit einer Referenzhäufigkeitsverteilung in Gestalt der logarithmischen Normalverteilung vorgenommen. Zwar ist es aus der DE-A-27 19 341 bekannt, zur Bestimmung der Rauhigkeit der Oberfläche der menschlichen Haut die Häufigkeitsverteilung dieses Parameters für die Auswertung heranzuziehen, doch werden dazu die an verschiedenen Zeiten und Orten gewonnenen Ergebnisse von Messungen an einem grossen Probandenkreis herangezogen, während bei der Erfindung die Auswertung anhand der bei einem Probanden über einen bestimmten ausgewählten Körperbereich im wesentlich gleichzeitig ermittelten Messwerte erfolgt, d.h. die logarithmische Referenz-Normalverteilung wird ebenfalls aus den erfassten Messwerten des jeweiligen Probanden gewonnen. Vorzugsweise wird als Körperbereich wegen der einfachen Verfügbarkeit die Haut des Probanden herangezogen, wobei die elektrische Leitfähigkeit der Haut oder deren Strahlungsintensität als physiologische Kenngrösse genommen werden kann. Die Erfindung ist jedoch weder auf derartige spezielle physiologische Kenngrössen noch auf den Körperbereich "Haut" beschränkt. Vielmehr ist das erfindungsgemässe Verfahren grundsätzlich auch auf andere Kenngrössen und andere geeignete innere oder äussere Körperbereiche anwendbar.

Die Erfindung macht sich die Tatsache zunutzen, dass nach den Regeln der Statistik Parameter gleich welcher Art immer einer bestimmten Häufigkeitsverteilung folgen (vgl. L. Sachs: Statistische Auswertungsmethoden, 2. Auflage, Springer Verlag Berlin 1969, S. 105-106). Unter "Häufigkeitsverteilung" wird dabei die Wahrscheinlichkeitsfunktion $p(x)$ verstanden, die die Häufigkeit oder Wahrscheinlichkeit angibt, einen bestimmten Messwert $x$ bei einem beliebigen Prüfobjekt anzutreffen, wobei $x$ die gesamte verfügbare Werteskala umfassen kann.

Die physiologischen Kenngrössen von Lebewesen, wie beispielsweise Körpergrösse, Blutdruck, Medikamentenverträglichkeit etc., sind darüber hinaus stets nach einer logarithmischen Normalverteilung gehäuft. Als Ursache hierfür wird ein multiplikatives Gestaltungsprinzip angenommen (vgl. z.B. auch: H. Gebelein und H.J. Heite, Klin. Wschr. 28 (1959), S. 41). Es wurde im Rahmen der erfindungsgemässen Versuche weiter festgestellt, dass die logarithmische Normalverteilung nicht nur für eine bestimmte Kenngrösse bei Messungen an einer Vielzahl von Individuen vorliegt, sondern auch bei einem einzigen gesunden Individuum, wenn die betreffende Kenngrösse an einer hinreichend grossen Anzahl von Messwerten des Individuums gemessen wird. "Hinreichend" in diesem Zusammenhang bedeutet, dass sich bei weiterer Vergrösserung der Anzahl der Messwerte an der resultierenden Häufigkeitsverteilung keine signifikante Änderung mehr ergibt.

Die ideale Log-Normal-Verteilung solcher Messwerte, die an einem einzelnen Probanden abgenommen werden können, liegt nur dann vor, wenn das ideale "multiplikative Gestaltungsprinzip" - d.h. das raumzeitliche Zusammenwirken aller Untereinheiten im Sinne einer idealen Organisation - erfüllt ist. Folglich kann

2

anhand des Vergleiches der gemessenen Häufigkeitsverteilung oder einer durch geeignete Transformation der Messwerte bestimmten Häufigkeitsverteilung mit der logarithmischen Normalverteilung eine eindeutige Klassifizierung des "ganzheitlichen" Zustandes bezogen auf den Zustand einer idealen biologischen Organisation gewonnen werden. Weitere diesbezügliche Aussagen lassen sich treffen, wenn gemäss anderer Weiterbildungen der Erfindung zusätzlich aus dem Vergleich die Abweichungen gleicher Ordnung, z.B. die relativen Unterschiede der Momente erster bis n-ter Ordnung, bestimmt werden und/oder die Änderung der Häufigkeitsverteilung mit der Zeit ermittelt und einer Korrelationsanalyse unterzogen wird. Die zeitliche Entwicklung der Häufigkeitsverteilung beschreibt das dynamische Verhalten des der Messung zugrunde liegenden Netzes interner Abhängigkeiten. Die Korrelationsanalyse (z.B. Faktorenanalyse) erlaubt bei bekannter Zuordnung der Messwerte zu ihren bestimmten Hautarealen eine dynamische Beschreibung interner Zusammenhänge zwischen den Hautarealen. Sie schliessen alle Wechselbeziehungen mit den Organen ein.

Daraus folgt, dass im Sinne der Erfindung ein Proband "ganzheitlich" als "gesund" gelten kann, wenn seine Verteilungsfunktion $p(x)$ nicht signifikant von $p_n(x)$ abweicht, wobei $p(x)$ die gemessene Verteilungsfunktion und $p_n(x)$ die ideale Verteilungsfunktion bei einem gesunden Individuum bedeuten. Diese Verteilungsfunktion $p_n(x)$ ist eine logarithmische Normalverteilung und kann erfindungsgemäss aus den Messwerten des Probanden ermittelt werden, d.h. es ist nicht erforderlich, die Normalverteilung als empirische Funktion aus den Messwerten einer Vielzahl von gesunden Probanden zu ermitteln.

Umgekehrt lässt sich der "Krankheitszustand" in diesem "ganzheitlichen" Sinne durch die systematisch (und vollständig) aufgelisteten Abweichungen zwischen den Funktionen $p(x)$ und $p_n(x)$ definieren. Ein wesentlicher Vorteil des erfindungsgemässen Verfahrens ist mithin, dass auf eine empirische Messwerterfassung an einer Vielzahl von Probanden nicht zurückgegriffen werden braucht, sondern die für den individuellen Probanden zutreffende ideale Verteilungsfunktion unmittelbar aus den gemessenen Werten errechnet werden kann und mit der wirklichen Häufigkeitsverteilung verglichen wird.

Bezüglich der Vorrichtung zur Durchführung des Verfahrens nach der Erfindung wird auf den Patentanspruch 8 verwiesen. Die Vorrichtung ermöglicht eine rasche Erfassung der Messwerte und deren Auswertung an Ort und Stelle. Aufgrund ihres unkomplizierten Aufbaues erfordert sie für ihre Bedienung keine Spezialkenntnisse und kann in preisgünstiger Weise realisiert werden.

Die Erfindung wird nachfolgend anhand eines Beispiels und der Zeichnung näher erläutert. Es zeigen:

Fig. 1a, 1b die Häufigkeitsverteilung der Leitfähigkeitswerte der Haut eines Patienten vor Behandlung (Fig. 1a) und nach Behandlung (Fig. 1b) im Vergleich zur logarithmischen Normalverteilung mit jeweils gleichen Mittelwerten und Varianzen.

Fig. 2a, 2b das Verhältnis der Momente r-ter Ordnung (r = 1...6) bei logarithmischer Normalverteilung und gemessener Verteilung vor Behandlung (Fig. 2a) und nach Behandlung (Fig. 2b),

Fig. 3 ein Blockdiagramm einer Vorrichtung zur Erfassung der Hautleitfähigkeit und zur Verarbeitung der gewonnenen Messwerte gemäss einer Ausführungsform der Erfindung,

Fig.4 in geschnittener Ansicht den Sensorteil einer Sensoranordnung der Vorrichtung nach Fig. 3, und

Fig. 5 die Sensoranordnung nach Fig. 3 in Unteransicht.

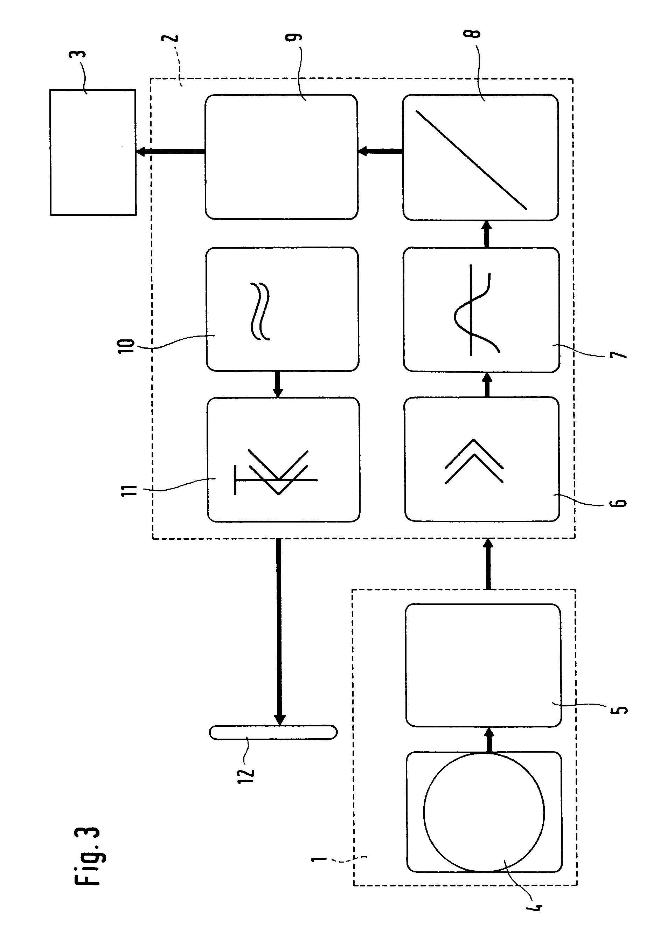

Mit Bezugnahme zunächst auf Fig. 3-5 wird nachfolgend eine Ausführungform einer Vorrichtung oder eines Gerätes zur Durchführung des erfindungsgemässen Verfahrens anhand einer Messung der elektrischen Leitfähigkeit der Haut eines Patienten beschrieben. Die Vorrichtung umfasst gemäss Fig. 3 eine Sensoranordnung 1, eine Signalverarbeitungseinrichtung 2 und eine Rechnereinheit 3.

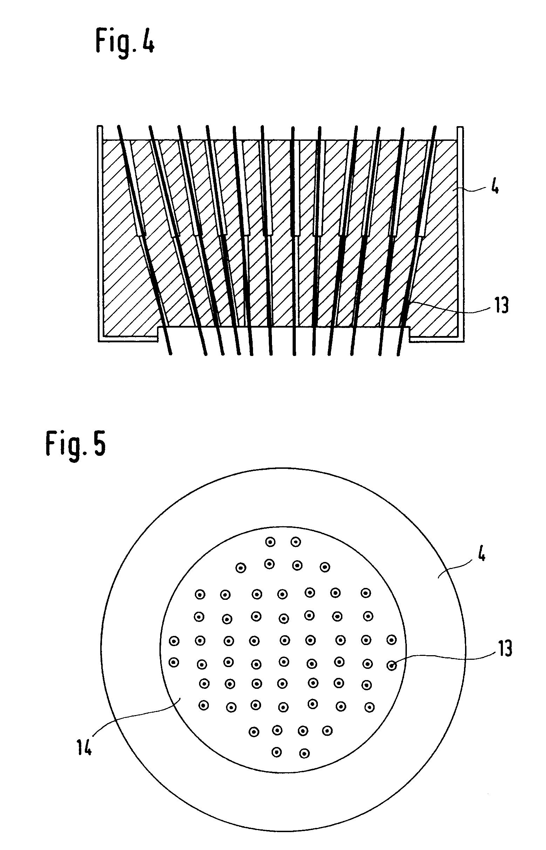

Bei der Sensoranordnung 1 kann es sich um eine Vielkanalelektrode, bestehend aus einem Sensorteil 4 und einem Abtast- oder Scannerteil 5, handeln. Der Sensorteil 4 ist in Fig. 4 und 5 näher gezeigt und umfasst eine Vielzahl von in einem Basisteil längsverschieblich gehaltenen nadelförmigen Elektroden oder Sensorelementen 13 ähnlich bekannten Akupunkturnadeln. Jedem Sensorelement 13 ist eine Vorspannfeder zugeordnet, die das Sensorelement in die in Fig. 4 gezeigte Ausganglage vorspannt, bei der die freien aus dem Basisteil herausragenden Enden der Sensorlemente 13 auf einer Ebene liegen, die flach oder entsprechend der Wölbung eines zu erfassenden Körperbereiches, z. B. der Hand, eines Probanden gekrümmt sein kann. Die Vorspannung der Sensorelemente 13 bewirkt, dass diese auf die Hautoberfläche einen definierten Druck ausüben, wenn sie damit in Anlage gebracht werden. Eine "hinreichende" Anzahl von Sensorelementen 13 ist vorgesehen. Es wurde festgestellt, dass eine Zahl von 50-150, z.B 60, Sensorelementen 13 hinreichend im eingangs erwähnten Sinne ist.

Die Sensorelemente 13 sind ferner über eine definierte, z.B. kreisförmige Messfläche 14 des Sensorteiles 4 verteilt. Der Abtast- oder Scannerteil 5 der Sensoranordnung 1, bei dem es sich um eine dem Fachmann grundsätzlich bekannte Bauart handeln kann, dient zum sukzessiven Abtasten der einzelnen Sensorlemente 13 und liefert für die an den einzelnen Sensorlementen 13 abgegriffenen Leitfähigkeitswerte kennzeichnende Signale an die Verarbeitungseinrichtung 2. Bei den Messwerten kann es sich z.B. um die "Zeigerabfälle" bei Methoden der heute üblichen "Elektroakupunktur" handeln, sobald die Messelektrode an der Messstelle mit konstantem Anpressdruck angebracht ist und ein Maximalwert zugrunde gelegt wird.

Die Signalverarbeitungseinrichtung 2 umfasst einen Verstärker 6 zur Verstärkung der einzelnen von der Sensoranordnung 1 ausgegebenen Signale. Der Ausgang des Verstärkers 6 liegt an einem Bypass-Filter 7 an, der bewirkt, dass eventuell vorhandene Störsignale aus den Messignalen ausgefilter werden. Die gefilterten Messignale werden anschliessend einem AD-Wandler 8 zugeführt. Die digitalen Ausgangssignale des AD-Wandlers 8 werden an einer Schnittstelle 9 der Signalverarbeitungseinrichtung 2 an eine Rechnereinheit 3 weitergegeben. Die Rechnereinheit 3 erhält auf diese Weise verstärkte und von Störeffekten befreite digitale Signale, die den mittels der Sensoranordnung 1 ermittelten Messignalen entsprechen.

Ferner umfasst die Signalverarbeitungseinrichtung 2 eine Einrichtung zum Anlegen einer definierten Referenzwechselspannung an einer geeigneten Stelle des Körpers des Probanden. Falls die Messwerterfassung an einer Seite der Hand des Probanden erfolgt, ist eine geeignete Messtelle für das Anlegen der Referenzspannung die andere Handseite. Die Einrichtung zum Anlegen der Referenzspannung umfasst einen Spannungsgenerator 10, dessen Ausgang über einen regelbaren Verstärker 11 an eine geeignete Handelektrode 12 geliefert wird.

Die Rechnereinheit 3 ermittelt aus den von der Verarbeitungseinrichtung 2 abgegebenen Signalen, die den erfassten Messwerten des Probanden entsprechen, zunächst die für den Probanden zutreffende logarithmische Normalverteilung $p_n(x)$, d.h. die für den Probanden ideale Verteilungsfunktion, und ferner die reale Verteilungsfunktion p(x). Die logarithmische Normalverteilung ist diejenige, die den gleichen Mittelwert x und die gleiche Streuung $\sigma$ wie die gemessene Verteilung p(x) hat. Anhand der Abweichungen zwischen p(x) und $p_n(x)$ ist eine Aussage über Art und Umfang der bestehenden gesundheitlichen Probleme möglich.

Die Rechnereinheit 3 ermittelt ferner andere für die Aussage über den Gesundheitszustand des Probanden kennzeichnende Grössen, wie z.B. das Verhältnis der Momente r-ter Ordnung der logarithmischen Normalverteilung zur gemessenen Häufigkeitsverteilung. Das Ergebnis der Berechnungen kann auf einem Bildschirm und/oder als Ausdruck in Form von Diagrammen oder in Tabellenform dargestellt werden. Die Rechnereinheit 3 übernimmt ferner die Lokalisation und Berechnung des maximalen Leitfähigkeitswertes innerhalb der gemessenen Matrix.

Nachfolgend wird anhand eines Rechenbeispieles die Berechnung der gemessenen Verteilungsfunktion p(x) und der logarithmischen Normalverteilung $p_n(x)$ erläutert. Bei den in dem Rechenbeispiel erwähnten Zahlenwerte handelt es sich um diejenigen, die in Tabelle 1 wiedergegeben sind.

Rechenbeispiel

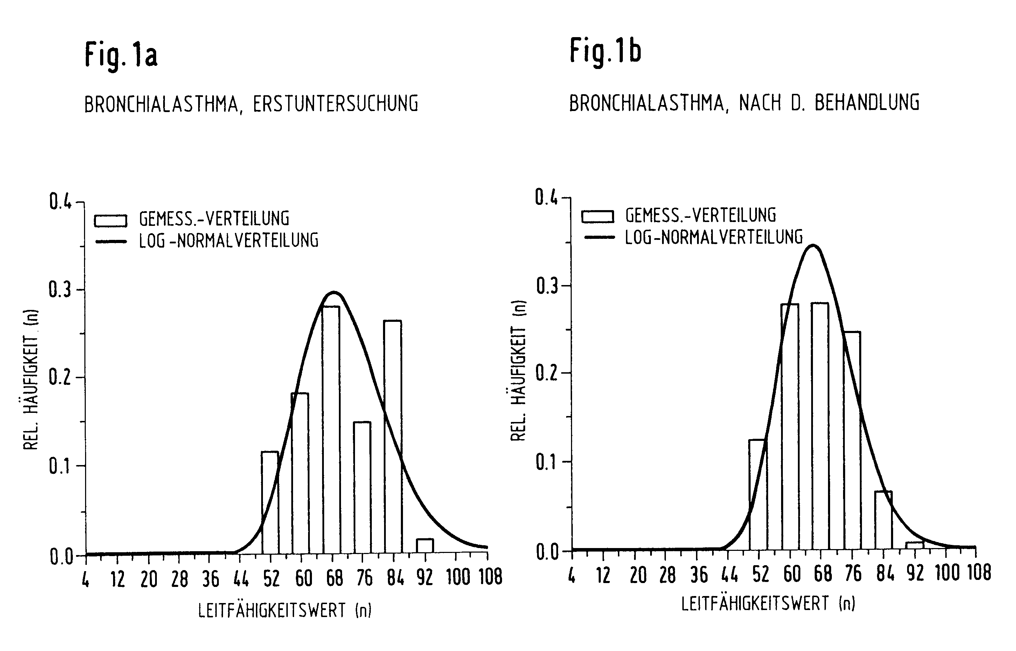

1. Einteilung der Häufigkeitswerte in n-Klassen, wobei hier n = 14 genommen wurde. Die Klassenmitten werden über den ganzen Messbereich (wie in Tab. 1 angegeben) mit 4, 12, 20, 28,...,108 in 8-er Schritte angegeben (wie die x-Achse der Fig. 1a, b zeigt). Im folgenden werden diese Werte als $K_{mitte}(i)$, mit i = 1,...,14 bezeichnet. Beispielsweise ist $K_{mitte}(2) = 12$, $K_{mitte}(3) = 20$.

2. Berechnung der gemessenen Verteilung p(x)

a) Berechnung der Summe, der in der Tabelle 1 angegebenen Häufigkeitswerte (p(x)). Als Beispiel werden die Werte vor der Behandlung genommen.

Summe im folgenden N genannt:

$$N = \sum_{i=1}^{k=14} P(x_i)$$

Also N = 0 + 14 + 22 + 34 + 18 + 32 + 2 + 0 = 122.

Die Häufigkeitswerte P(x) werden durch die Summe N dividiert.

$$\frac{0}{122} = 0, \quad \frac{14}{122} = 0.115, \quad \frac{22}{122} = 0.18, \quad \frac{34}{122} = 0.279, \quad \frac{18}{122} = 0.148,$$

$$\frac{32}{122} = 0.262, \quad \frac{2}{122} = 0.016, \quad \frac{0}{122} = 0.$$

Als Formel:

4

$$p(x_i) = \frac{1}{N}P(x_i) = P_i$$

Diese gemessene Verteilung wird in der Grafik als Balken dargestellt.

3. Berechnung der Log-Normalverteilung
   - Berechnung Mittelwert $\bar{x}$ und Streuung $\sigma$:

$$\bar{x} \;=\; \frac{1}{N}\sum_{i=1}^{k} P(x_i) * Kmitte(i)$$

$$\sigma \;=\; \sqrt{\frac{1}{N-1}\sum_{i=1}^{k}(Kmitte(i) - \bar{x})^2 P(x_i)}$$

Beispiel:

$$\bar{x} = \frac{1}{122}(14*52 + 22*60 + 34*68 + 18*76 + 32*84 + 2*92) = 70.49.$$

$h\sigma$ = (52-70.49)²*14 + (60-70.49)²*22 + (68-70.49)²*34 + (76-70.49)²*18 + (84-70.49)²*32 + (92-70.49)²*2

$$\sigma \;=\; \sqrt{\frac{1}{121} * h\sigma}$$

Hilfsgrößen:

$$\kappa = \sqrt{\ln\left(\frac{\sigma^2}{\bar{x}^2} + 1\right)} = 0.156.$$

$$\mu = \ln\bar{x} - \frac{\ln\sigma^2}{2} = 4.243 \quad\cdot$$

Log-Normalverteilung

$$p_n(x_i) = \frac{1}{\sqrt{2\pi \ln\sigma\, Kmitte(i)}}\exp\left(-\frac{1}{2}\left(\frac{\ln Kmitte(i) - \mu}{\kappa}\right)^2\right)$$

Bsp. für die Klassenmitte 68:

$$p_n(68) = \frac{1}{\sqrt{2 * \pi * 0.156 * 68}} \exp\left(-\frac{1}{2}\left(\frac{4.219 - 4.243}{0.156}\right)^2\right) = 0.121$$

Dann werden alle $p_n(x_i)$-Werte über alle i aufsummiert und durch die Gesamtsumme dividiert. Die Gesamtsumme

$$\sum_{i=1}^{k=14} p_n(x_i) = 0.412,$$

so daß z.B. an der Stelle 68 nicht 0.121, sondern, nach dieser Normierung

$$p_n(68) = \frac{0.121}{0.412} = 0.294$$

ist.

Beispiel

Bei einem Patienten mit schwerem Bronchialasthma wurden an 112 Messtellen der Haut die elektrischen Leitfähigkeitswerte ermittelt und die relative Häufigkeit der Werte über einer Skala von 0 bis 100 aufgetragen.

Die Häufigkeiten, mit der Werte in den verschiedenen Skalenbereichen gemessen wurden, sind in Tabelle 1 für n = 8 Messintervalle aufgelistet. Die linke Spalte bezieht sich auf die Werte vor der Behandlung, während die rechte Spalte die Werte nach relativ erfolgreicher Behandlung (der Patient hat weniger Beschwerden) wiedergibt.

Aus den Daten selbst ist weder ein objektives Kriterium für den Gesundheitszustand des Patienten vor der Behandlung, noch für das Mass der Besserung nach der Behandlung zu entnehmen. Prüft man dagegen die Häufigkeiten p(n), bestimmte Leitfähigkeitswerte n zu erhalten, auf ihre Übereinstimmungen mit der logarithmischen Normalverteilung (in Fig. 1a und 1b durch ausgezogene Linien wiedergegebene Kurve), so stellt man fest:

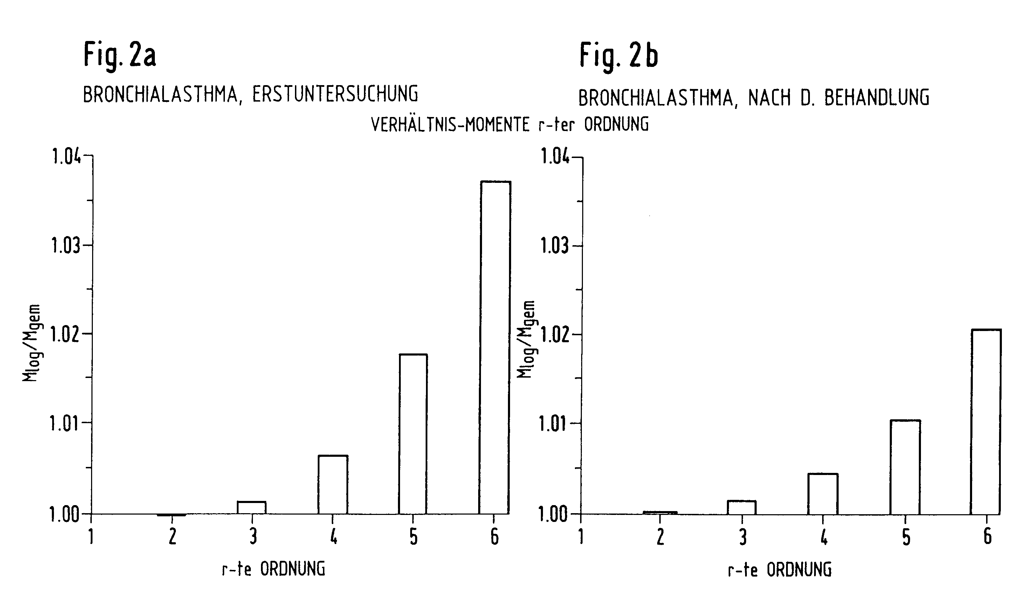

1) Vor der Behandlung ergeben sich signifikante Abweichungen von der Normalverteilung (Fig. 1a) wie auch in den Abweichungen der Momente dritter und höherer Ordnung (Fig. 2a), definiert als

$$\left(m^r = \sum_{i=1}^{N} p(n_i) \cdot (n_i - \bar{n})^r\right)$$

Dies indiziert, dass der Patient nicht gesund ist. Art und Schwere der Erkrankung sind in dieser Projektion als Art und Mass der Abweichung von der logarithmischen Normalverteilung erkennbar.

2) Nach der Behandlung wird eine wesentlich bessere Übereinstimmung sowohl mit der logarithmischen Normalverteilung (Fig .2b) als auch eine geringere Abweichung der Momente höherer Ordnung von den idealen Momenten aus der Normalverteilung erkennbar. Die Kurven sind dabei so transformiert, dass die Momente erster und zweiter Ordnung (Mittelwerte und Varianzen) der idealen und der gemessenen Verteilung übereinstimmen.

EP 0 538 739 B1

## Tabelle 1: Gemessene Häufigkeiten von Leitfähigkeitswerten an 112 Hautpunkten in einem zugeordneten Messbereich 0 bis 100 bei einem Probanden mit Bronchial-Asthma.

| | vor | Behandlung | nach |
|---|---|---|---|
| Messbereich | Häufigkeiten | | Häufigkeiten |
| 0 – 48 | 0 | | 0 |
| 48 – 56 | 14 | | 15 |
| 56 – 64 | 22 | | 34 |
| 64 – 72 | 34 | | 34 |
| 72 – 80 | 18 | | 30 |
| 80 – 88 | 32 | | 8 |
| 88 – 96 | 2 | | 1 |
| 96 – 112 | 0 | | 0 |

Vorausgehend wurde die Erfindung anhand der Messung der elektrischen Leitfähigkeit der Haut als physiologische Kenngrösse beschrieben. Werden andere Kenngrössen herangezogen, ist die Vorrichtung entsprechend zu modifizieren. Als Kenngrösse kann z.B. die Intensität der Abstrahlung der Haut im Infratrot- oder optischen Bereich dienen. In diesem Fall werden vorzugsweise berührungslose Sensorelemente in einer Anordnung und Anzahl entsprechend den nadelförmigen Sensorelementen der vorbeschriebenen Ausführungsform vorgesehen. Andere Erfassungseinrichtungen für die physiologischen Kenngrössen können in Gestalt von Gitter-, Roll- oder Bürstenelektroden ausgebildet sein. Es wurde ,ferner insbesondere die bevorzugte Beurteilung des ganzheitlichen Gesundheitszustandes eines Probanden anhand des Vergleiches der realen Verteilungsfunktion mit der idealen, d.h. logarithmischen Normalverteilung der am Probanden ermittelten Messwerte beschrieben. Es versteht sich jedoch, dass durch die Erfindung auch ein Vergleich anhand einer Referenzhäufigkeitsverteilung von Daten umfasst ist, die für die betreffende physiologische Kenngrösse aus Messungen bei einer Anzahl von gesunden Individuen ermittelt wurden.

**Patentansprüche**

1. Verfahren zur Ermöglichung einer Aussage über den Gesundheitszustand eines Lebewesens anhand eines Vergleiches einer ausgewählten physiologischen Kenngrösse des Lebewesens mit einer entsprechenden für den gesunden Zustand kennzeichnenden Referenzkenngrösse, dadurch gekennzeichnet, dass die ausgewählte physiologische Kenngrösse mittels einer Mehrkanal-Sensoreinheit, welche eine Vielzahl von über einen bestimmten Flächenbereich verteilten Sensorelementen aufweist, an einer der Vielzahl der Sensorelemente entsprechenden Vielzahl von Messstellen erfasst, mittels Methoden der Statistik aus den von der Sensoreinheit abgegebenen und den Messwerten der ausgewählten physiologischen Kenngrösse entsprechenden Signalen die statistische Verteilung und die logarithmische Normalverteilung der Signalpegel bestimmt, und eine Ermittlung der Abweichungen der statistischen Verteilung von der logarithmischen Normalverteilung der Signalpegel vorgenommen wird, wobei die logarithmische Normalverteilung als Referenzkenngrösse genommen wird.

2. Verfahren nach Anspruch 1, dadurch gekennzeichnet, dass als Körperbereich ein Bereich der Haut des Lebewesens herangezogen wird.

3. Verfahren nach Anspruch 1, dadurch gekennzeichnet, dass die zu erfassende physiologische Kenngrösse die Leitfähigkeit der Haut bei Anliegen eines bestimmten elektrischen Potentials ist.

**4.** Verfahren nach Anspruch 3, dadurch gekennzeichnet, dass die zeitliche Änderung der Leitfähigkeit entsprechend Methoden der Elektroakupunktur ermittelt wird.

**5.** Verfahren nach Anspruch 1, dadurch gekennzeichnet, dass die zu erfassende physiologische Kenngrösse die Strahlungsintensität, insbesondere im optischen oder Infratrotbereich, der Haut ist.

**6.** Verfahren nach einem der vorhergehenden Ansprüche, dadurch gekennzeichnet, dass aus dem Vergleich die Abweichungen gleicher Ordnung bestimmt werden.

**7.** Verfahren nach einem der vorhergehenden Ansprüche, dadurch gekennzeichnet, dass ferner die Änderung der Häufigkeitsverteilung mit der Zeit ermittelt und einer Korrelationsanalyse unterzogen wird.

**8.** Vorrichtung zur Ermöglichung einer Aussage über den Gesundheitszustand eines Lebewesens nach Anspruch 1, gekennzeichnet durch eine Mehrkanal-Sensoreinheit (1) zur Erfassung einer ausgewählten physiologischen Kenngrösse des Lebewesens an einer Vielzahl von über einen bestimmten Flächenbereich des Körpers verteilten Messtellen und zur Abgabe von entsprechenden Signalen, eine Einrichtung (2,3) zum Verarbeiten der von der Mehrkanal-Sensoreinheit abgegebenen Signale, und eine Einrichtung, um aus den von der Verarbeitungseinrichtung abgegebenen Signalen mittels Methoden der Statistik die tatsächliche Häufigkeitsverteilung und die logarithmische Normalverteilung der den Signalen entsprechenden Messwerte der erfassten physiologischen Kenngrösse zu berechnen.

**9.** Vorrichtung nach Anspruch 8, dadurch gekennzeichnet, dass die Mehrkanal-Sensoreinheit (1) eine Vielzahl von über einen definierten Flächenbereich verteilt angeordneten Sensorelementen (13) sowie eine Einrichtung (15) zum sukzessiven Abtasten der Sensorelemente umfasst.

**10.** Vorrichtung nach Anspruch 9, dadurch gekennzeichnet, dass die Sensorelemente nadelartige Elemente (15) ähnlich Akupunkturnadeln umfassen.

**11.** Vorrichtung nach Anspruch 8 oder 9, dadurch gekennzeichnet, dass die Mehrkanal-Sensoreinheit (1) Sensorelemente zur berührungslosen Messwerterfassung aufweist.

**12.** Vorrichtung nach Anspruch 8 oder 9, dadurch gekennzeichnet, dass die Mehrkanal-Sensoreinheit (1) Gitter-, Roll- oder Bürstenelektroden zur Erfassung der physiologischen Kenngrösse umfasst.

**Claims**

**1.** A method of permitting an indication as to the health condition of a living creature on the basis of a comparison of a selected physiological characteristic of the living creature to a corresponding reference characteristic of the healthy condition characterized by said selected physiological characteristic being detected by means of a multi-channel sensor unit having a plurality of sensor elements distributed over a predetermined surface area, at a plurality of measurement points corresponding to said plurality of sensor elements, determining by means of statistical methods the statistical distribution and the logarithmic normal distribution of the signal levels of the signals output by said sensor unit and corresponding to the measurement values of said selected physiological characteristic, performing an establishing of the departures of the statistical distribution from the logarithmic normal distribution of said signal levels, whereby said logarithmic normal distribution being taken as a reference characteristic.

**2.** A method according to claim 1, wherein a region of the skin of the living creature is employed as the body region.

**3.** A method according to claim 1, wherein the physiological characteristic to be detected is the conductivity of the skin to which a specific electric potential is applied.

**4.** A method according to claim 3, wherein the change with time in the conductivity is established according to methods of electroacupuncture.

5. A method according to claim 1, wherein the physiological characteristic to be detected is the radiation intensity of the skin, particularly in the optical or infrared range.

6. A method according to any of the preceding claims, wherein the deviations of the same order are determined from the comparison.

7. A method according to any of the preceding claims, wherein furthermore the change in the statistical distribution is established as a function of time and subjected to a correlation analysis.

8. A device for permitting an indication as to the health condition of a living creature according to claim 1, characterized by a multi-channel sensor unit (1) for detecting a selected physiological characteristic of the living creature at a plurality of measurement points distributed over a defined body region and for outputting corresponding signals, a means (2,3) for processing the signals output by said multi-channel sensor unit, and a means for calculating from the signals output by said signal processing means the actual statistical distribution and the logarithmic normal distribution of the signal-related measured values of the physiological characteristic obtained.

9. A device according to claim 8, wherein said multi-channel sensor unit (1) includes a plurality of sensor elements (13) distributed over a defined surface area and means (15) of successively scanning said sensor elements.

10. A device according to claim 9, wherein said sensor elements include needle-shaped elements (15) similar to acupuncture needles.

11. A device according to claim 8 or 9, wherein said multi-channel sensor unit (1) comprises sensor elements for obtaining the measured values by proximity.

12. A device according to claim 8 or 9, wherein said multi-channel sensor unit (1) includes grid, roller or brush-type electrodes to obtain the physiological characteristic.

**Revendications**

1. Procédé permettant l'établissement d'un rapport sur l'état de santé d'un être vivant en s'appuyant sur une comparaison d'un paramètre physiologique choisi de l'être vivant avec un paramètre de référence correspondant, caractéristique de l'état de bonne santé, caractérisé en ce que le paramètre physiologique choisi est mesuré au moyen d'une unité de détection à canaux multiples qui présente une pluralité de capteurs répartis sur une portion de surface déterminée, en une pluralité de points de mesure correspondant à la pluralité de capteurs, en ce que la distribution statistique et la distribution normale logarithmique des niveaux de signal sont déterminées par des méthodes de statistique à partir des signaux délivrés par l'unité de détection et correspondant aux valeurs mesurées du paramètre physiologique choisi et en ce qu'une analyse des écarts de la distribution statistique et de la distribution normale logarithmique des niveaux de signal est effectuée, la distribution normale logarithmique étant prise comme paramètre de référence.

2. Procédé selon la revendication 1, caractérisé en ce que la partie du corps utilisée est une zone de la peau de l'être vivant.

3. Procédé selon la revendication 1, caractérisé en ce que le paramètre physiologique à mesurer est la conductibilité électrique de la peau lors de l'application d'un potentiel électrique déterminé.

4. Procédé selon la revendication 3, caractérisé en ce que la variation dans le temps de la conductibilité est déterminée selon des méthodes de l'électro-acupuncture.

5. Procédé selon la revendication 1, caractérisé en ce que le paramètre physiologique à mesurer est l'intensité de rayonnement de la peau, notamment dans le domaine optique ou infrarouge.

6. Procédé selon l'une quelconque des revendications précédentes, caractérisé en ce que les écarts de même ordre sont déterminés à partir de la comparaison.

**7.** Procédé selon l'une quelconque des revendications précédentes, caractérisée en ce que de plus les variations dans le temps de la distribution de fréquence sont déterminées et soumises à une analyse de corrélation.

**8.** Dispositif permettant l'établissement d'un rapport sur l'état de santé d'un être vivant selon la revendication 1, caractérisé par une unité de détection à canaux multiples (1) pour la mesure d'un paramètre physiologique choisi de l'être vivant en une pluralité de points de mesure répartis sur une portion de surface déterminée du corps et pour la délivrance de signaux correspondants, un appareillage (2,3) pour le traitement des signaux délivrés par l'unité de détection à canaux multiples, et un appareillage pour calculer au moyen de méthodes de statistique, à partir des signaux délivrés par l'appareillage de traitement, la distribution de fréquence réelle et la distribution normale logarithmique des valeurs mesurées, correspondant aux signaux, du paramètre physiologique mesuré.

**9.** Dispositif selon la revendication 8, caractérisé en ce que l'unité de détection à canaux multiples (1) comprend une pluralité de capteurs (13) répartis sur une portion de surface définie ainsi qu'un appareillage (15) pour l'exploration successive des éléments de capteur.

**10.** Dispositif selon la revendication 9, caractérisé en ce que les éléments de capteur comprennent des éléments en forme d'aiguilles (15) semblables aux aiguilles d'acupuncture.

**11.** Dispositif selon la revendication 8 ou 9, caractérisé en ce que l'unité de détection à canaux multiples (1) présente des éléments de capteur pour le relevé sans contact des valeurs de mesure.

**12.** Dispositif selon la revendication 8 ou 9, caractérisé en ce que l'unité de détection à canaux multiples (1) comprend des électrodes en forme de grille, de rouleau ou de brosse pour la mesure du paramètre physiologique.

# Fig.1a

BRONCHIALASTHMA, ERSTUNTERSUCHUNG

# Fig.1b

BRONCHIALASTHMA, NACH D. BEHANDLUNG

EP 0 538 739 B1

## Fig. 2a

BRONCHIALASTHMA, ERSTUNTERSUCHUNG

VERHÄLTNIS-MOMENTE r-ter ORDNUNG

## Fig. 2b

BRONCHIALASTHMA, NACH D. BEHANDLUNG

EP 0 538 739 B1

Fig. 3

EP 0 538 739 B1

## Fig. 4

## Fig. 5